# EUROPEAN PATENT APPLICATION

(11) **EP 2 269 681 A2**
(43) Date of publication of application: **05.01.2011**
(21) Application number: 10165242.8
(22) Date of filing: 08.06.2010
(51) Int. Cl.: A61M 16/16, A61M 16/10

(54) **Humidifier for humidifying a gas in a respiratory circuit**

(30) Priority: 30.06.2009 EP 09008514
(71) Applicant: Covidien AG, 8212 Neuhausen am Rheinfall (CH)
(72) Inventor: Zucchi, Giuseppe, 41039, San Possidonio (Modena) (IT); Resca, Daniele, 41038, San Felice sul Panaro (Modena) (IT)
(74) Representative: Lanzoni, Luciano

(57) **Abstract**

A humidifier for humidifying a gas in a respiratory circuit (20), the humidifier comprising an evaporator (1) physically arranged outside the respiratory circuit (20) and designed to receive a defined quantity of water, vaporize said defined quantity of water and supply the vaporized water to the respiratory circuit (20); the humidifier further including a water reservoir (2) which is connected to the evaporator (1) via a dosing system (30).

## Description

The invention relates to a humidifier for humidifying a gas in a respiratory circuit.

In respiratory therapy to patients who are unable to breathe on their own, the physiological function of natural humidification of inspired air performed by upper airways is bypassed, and through the ventilation, the dry air is channeled directly to the bronchi. In this situation the need to moisten the air supplied to the patient in order to simulate as close as possible the natural condition is highlighted.

Currently the active humidification of respiratory gases is performed through equipments described below.

A first known humidifier consists of a hot plate housing a reservoir of water. This reservoir is connected directly to the breathing circuit through a gas input and output port. The water content is heated from the heater underneath in order to saturate the air above of humidity, collected from the respiratory gas flow passing through the reservoir. The enrichment of moisture in respiration gas takes place by lapping the surface of the heated water. The volume of humidity saturated air corresponds to the maximum capacity of the reservoir (i.e. the space free of water). If the volume of inspiratory act is far greater than the one of the reservoir, there will be a much richer humidification at the beginning of the act and a poor moisture air content in the final portion.

A second known device consists of a heating system featuring a jacket housing the cylindrical water reservoir. The reservoir is made of a double wall, enclosing an hollow space, where the water to be evaporated is collected. The inner wall consists of a vapor permeable membrane that faces directly on the gas flow; the outer wall is made on aluminum. The heating envelopment, in contact with the outer wall made of aluminum, causes evaporation of the film water present in the interspace and then the vapor permeates the inner membrane towards the flow of respiratory gases. The device provides an uneven distribution of moisture during the inspiratory act.

In the above systems it is not possible to define exactly the amount of moisture supplied to each patient ventilation cycle, only an approximate value based on the temperatures involved can be calculated. Furthermore it is necessary to heat water in excess in order to have a quantity of vapor in the breathing circuit sufficient to the patient correct humidification. Because of the amount of water involved, these systems have a certain thermal inertia and therefore a slow response to sudden change in humidification settings and a considerable power demand.

A further system for instantaneous evaporation of water for humidification of respiratory gases is described in U.S. patent 4,038,980. The system described consists of an evaporation chamber that is inserted directly on the path of inspiration gases. In this system the water feeding system works non-synchron with the respiratory cycle with the result of having a always different moisture concentration for each respiratory act.

The known respiration humidifiers described above have the drawback that the humidification of the respiratory gas is substantially uncontrolled; it is obtained from the heating power set and/or the flow rate. It can happens either that the respiratory gas is supersaturated, or that the respiratory gas is humidified insufficiently. The technical purpose of the present invention is to overcome the drawbacks described above by providing a respiratory active humidification system which combines the use of an evaporation chamber, for continuous steam production, with a system for collection and release of humidity to maintain a uniform humidity level on patient inhaled gases.

It is another aim of the invention to provide a respiratory active humidification system which is accurately controllable to provide predetermined air temperature and relative humidity and which provides humidified air in a sterile condition.

The technical purpose and aim specified are substantially achieved by a humidifier comprising the technical features described in one or more of the claims below.

Further features and advantages of the present invention will become apparent from the description below of a preferred embodiment shown in the accompanying drawings, given purely by way of a nonlimiting example, in which:
- Figures 1 and 2 are schematic views of the humidifier according to the present invention;
- Figure 3 shows a view of the steam generation trend over time and the combination with the performance of respiratory gas flow.
- Figure 4 shows a schematic view of a respiratory circuit comprising a humidifier according to the present invention;
- Figure 5 shows a schematic view of a respiratory circuit according to the state of the art.

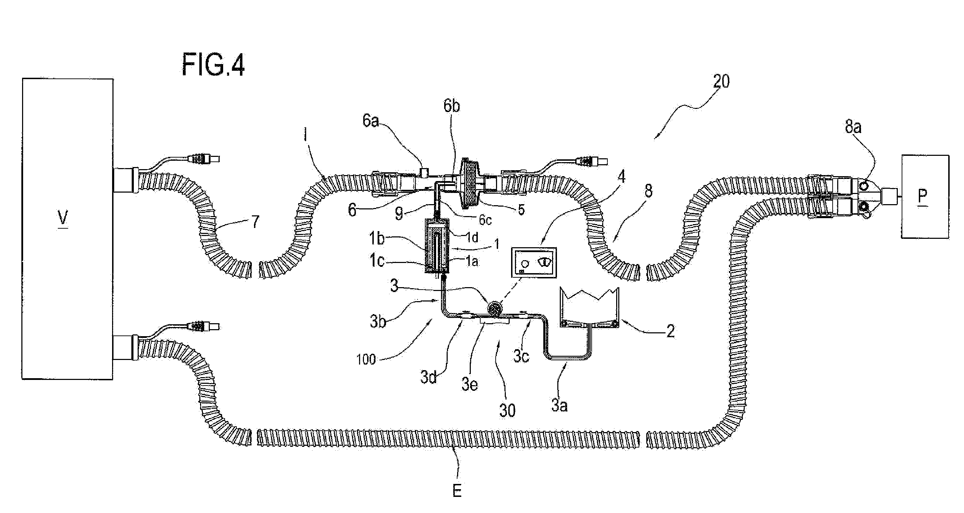

In the drawings, the numeral 20 denotes in its entirety a respiratory circuit interposed between a patient P and a breathing machine e.g. a ventilator V, illustrated only in Figure 4.

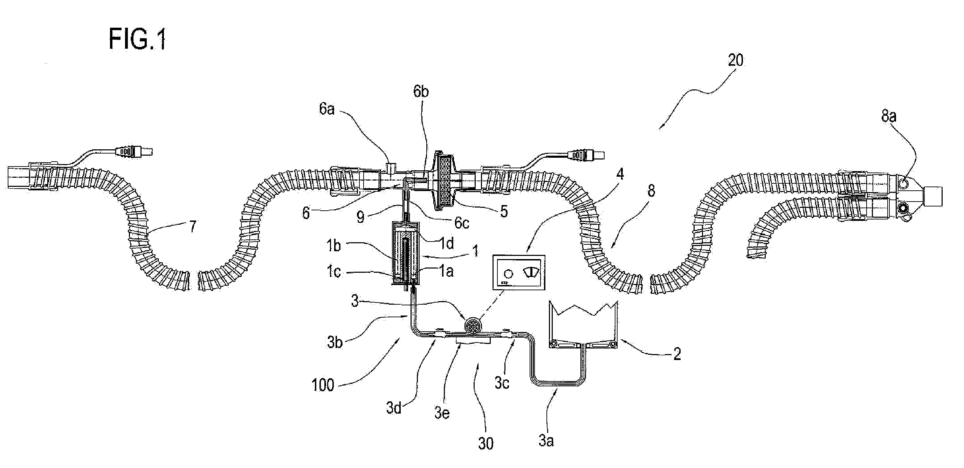

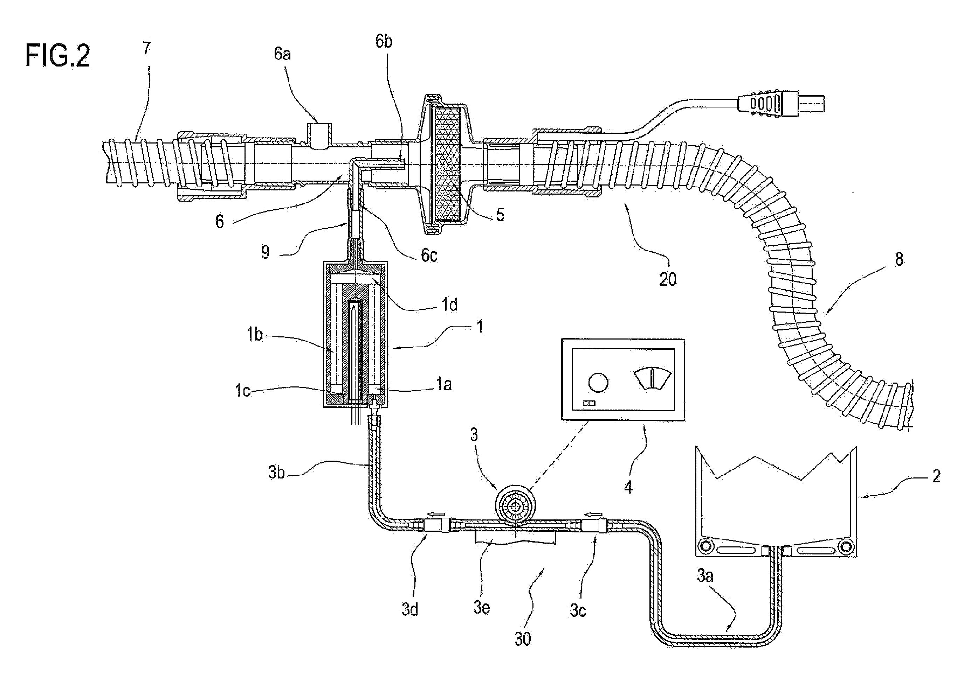

In particular, Figures 1 and 2 illustrate an inspiratory limb I of the respiratory circuit 20; Figure 4 illustrates the respiratory circuit 20 comprising both the inspiratory limb I and the expiratory limb E.

The respiratory circuit 20 comprises an humidifier 100, a first heated wire circuit 7, upstream to the humidifier 100 and a second heated wire circuit 8, downstream to the humidifier 100.

In this way the inspiratory limb I of the respiratory circuit 20 is divided into a first section upstream (defined at least in part by the first circuit 7), located between the ventilator and the humidifier 100, and a second section 8 downstream the humidifier 100 (defined at least in part by the second circuit 8), located between the humidifier 100 and the patient.

As illustrated in figure 1, the humidifier 100 comprises an evaporator 1, able to supply water vapour inside the respiratory circuit 20, a water reservoir 2 connected to the evaporator 1 and a dosing system 30 allowing the transfer of a defined quantity of water from the reservoir 2 to the evaporator 1.

The dosing system 30 comprises a motion movement mechanism 3, a deformable member 3e, connecting pipes 3a and 3b to connect the deformable member 3e respectively to the reservoir 2 and to the evaporator 1, two one-way valves 3c, 3d.

The two one-way valves 3c, 3d are included, one on the pipe 3a connecting the reservoir 2 to the deformable member 3e and another on the pipe 3b connecting the latter to the evaporation chamber 1 a. The humidifier 100 further comprises a regulating device 4 acting on the dosing system 30 to allow the physician to set up the water quantity to be sent to the evaporator 1 with aim to obtain the desired absolute humidity level in the gas inhaled by the patient.

The evaporator 1 comprises an evaporation chamber 1a located at a lower base, a central heating cartridge 1c and heated channels 1b distributed evenly around the cartridge 1 c.

The evaporator 1 further comprises, opposite to the evaporation chamber 1 a, an upper chamber 1d in which the superheated steam is collected.

The evaporation chamber 1a and the upper chamber 1d are connected by means of the cited heated channels 1 b.

As clearly shown in figure 2, according to a preferred embodiment of the present invention, a connector 6 is interposed between the first heated wire circuit 7 and the second heated wire circuit 8 of the inspiratory limb I of circuit 20.

Between the connector 6 and the second heated wire circuit 8 a humidity exchanger element 5 is interposed.

The connector 6 is made of material able to resist to the vapour high temperatures and presents a tubular body, a temperature probe port 6a and a connector port 6c, extending outside and able to connect the evaporator 1 to the connector 6 itself.

Into the connector 6 a vapour diffusion nozzle 6b is included, starting perpendicular to the connector 6 and coaxial to the same for at least the last portion of its tubular body, aimed to deliver vapour coming from the evaporator 1 directly on the humidity exchanger element 5. The nozzle 6b is connected to the evaporator 1 by means of the connector port 6c extending outside and of a connecting channel 9 better disclosed below.

The humidity exchanger element 5 is thus disposed along the inspiratory limb I of the respiratory circuit 20, downstream to the vapour diffusion nozzle 6b.

The humidity exchanger element 5 is preferably made of highly hygroscopic material.

This humidity exchanger element 5 is ideally able to collect all the vapour quantity produced in the expiratory phase (when it is not used), and made of thermal insulating material in order to avoid burns (due to the vapour high temperature) and prevent condensation caused by cooling.

As clearly disclosed in the present description and appended claims, the humidity exchanger element 5 defines an accumulator for collecting vaporized fluid and exchanging it with the gas flowing along the respiratory circuit 20.

The humidity exchanger element 5 can be equipped with water display/collection means (not shown) which can be useful in case of physician incorrect humidity level set up and an hazardous resistance to flow level could be reached.

As shown in figure 1, the first heated wire circuit 7 is positioned upstream to the humidifier 100 in order to pre-heat the ventilation gases and make them more eager of water (compared to colder gas).

Accordingly, the second heated wire circuit 8, positioned downstream to the humidifier 100, has the aim of maintaining the respiratory gases temperature (avoiding the humidity condensation) and is designed to make the gases reach the desired temperature (usually around 37°C). Said gases temperature is measured by means of a temperature probe, not illustrated, placed on a port 8a located near to the patient.

The humidifier 100 according to the present invention acts as follows. The physician sets up, by means of the regulating device 4, the humidification extent he desires to supply to the patient, selecting the water quantity to be delivered.

This regulations turn into a variation of the water quantity towards the evaporator.

The dosing system 30 withdraws water from the reservoir 2 and transfers it to the evaporation chamber 1a by means of connecting pipes 3a and 3b.

As described above, on the pipes 3a and 3b at least two one-way valves 3c, 3d are included, one on the tube 3a connecting the reservoir 2 to the dosing system 30 and another on the tube 3b connecting the latter to the evaporation chamber 1 a. These one-way valves 3c, 3d prevent the fluid from returning to the reservoir 2 even when back pressure is present into the evaporation chamber 1.

As shown in Figure 2, a feasible embodiment of the dosing system 30 consists of an eccentric motion movement mechanism 3 that raises or lowers over the water containing tube (i.e. the deformable member 3e) causing its deformation. The deformation results in a change in volume inside the tube. Under crushing, the pressure inside the tube increases causing the closure of the upstream valve 3c and simultaneously opening the downstream valve 3d. This results in a transfer of a defined volume of water from the pipe 3b to the evaporation chamber 1a. In the subsequent release of the tube deflection, the pressure inside the tube decreases then causing the closure of the downstream valve 3d and the opening of upstream valve 3c withdrawing water from the reservoir 2.

The precise amount of metered water flows into the evaporation chamber 1 a, distributing on a thin layer over the available area at the base of the chamber 1a itself. Then, by means of the high temperature of the chamber 1a, caused by the heating of the cartridge 1 c, the metered water it is instantly turned into steam.

The vapour formed by instantaneous water vaporization flows through the heated channels 1b distributed evenly around the central heating cartridge 1c. Through this constrained path the steam is further heated to a temperature exceeding 100° C which can advantageously reach 130°C.

The superheated steam is finally collected in the upper chamber 1d from where it is carried outside.

Advantageously, being the evaporation chamber 1a not disposable, the constant heating at high temperatures procures the sterilization of the chamber 1a itself.

Through a connecting channel 9 the steam is then transferred to the respiratory gases flowing into the inspiratory limb I of the respiratory circuit 20.

The connecting channel 9 is preferably made of heat resistant material (such as a silicone tube or connector) linked to the connector 6 through the port 6c. As already mentioned above, the connector 6 is defined by a small diameter pipe in order to maintain a constant flow of steam, ending at one side into the port 6c and on the other side in the vapour diffusion nozzle 6b, the two opposite ending parts of the pipe being substantially perpendicular.

Thus, the vapour diffusion nozzle 6b deflects the flow of steam on the humidity exchanger 5 direction by means of its last portion extending coaxial to both the tubular body of the connector 6 and the humidity exchanger 5, the latter advantageously presenting an axially symmetric shape.

Advantageously the humidity exchanger 5 comprises an HME (Heat and Moisture Exchanger) element or a combination of an HME element and a filter element (also called filter-HME).

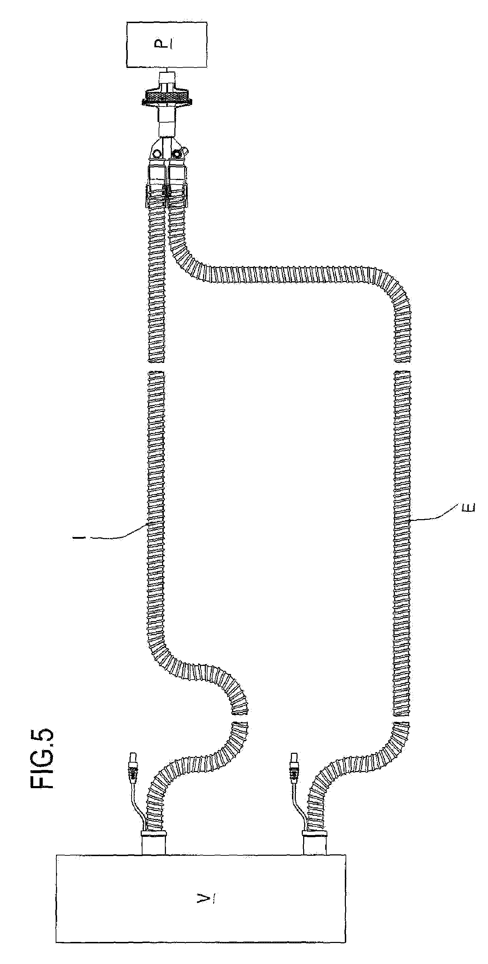

As shown in Figure 5 according to the state of the art, an HME is positioned downstream the Y connection of a respiratory circuit, in order to trap heat and humidity during exhalation and returning good portion of the same to the patient in inhalation phase.

The production of steam obtained by the system object of this invention is stable over time around the level predetermined by the action on the regulator 4.

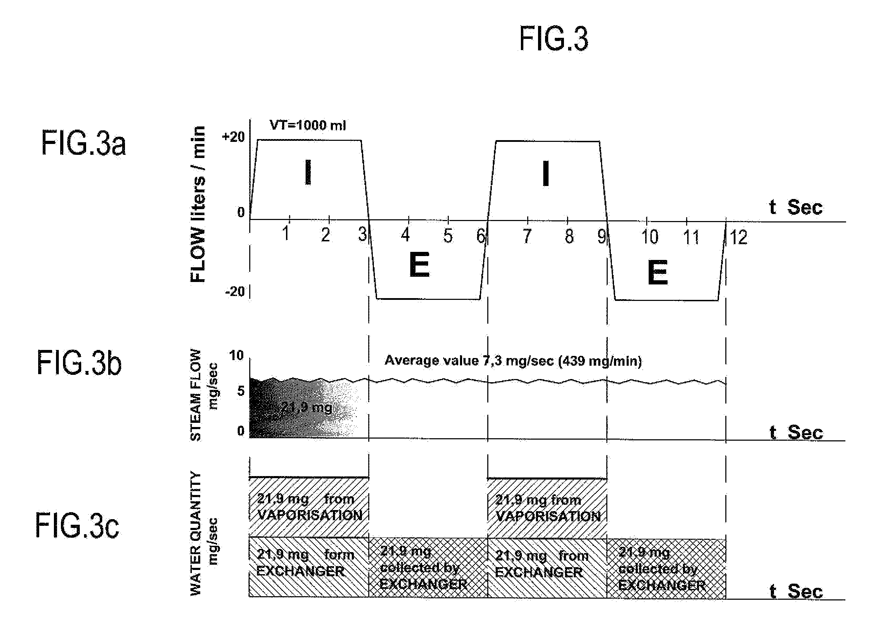

The view of the steam generation trend over time and the combination with the performance of respiratory gas flow is illustrated in Fig. 3 and detailed below.

Figure 3 a) is a trend of alternating inspiration/expiration ventilation cycle: each respiratory cycle consists of an inspiratory phase in which a positive volume of air is sent to the patient and an expiratory phase in which the same volume is returned from the patient to the ventilator. The result is a general sinusoidal pattern of the airflow.

Figure 3 b) is a trend of the steady flow of steam: the effect of continuous micro-dosing of water to the evaporation chamber results in a nearly constant flow of steam to the inside of the breathing circuit.

Figure 3 c) is a trend of steam quantity available in the circuit during ventilation: the amount of steam produced during the expiratory phase and gathered by the humidity exchanger element is once again available to the next inspiratory phase. Every inspiratory phase will always contain the same amount of steam.

The humidity exchanger element 5, consisting of high-efficiency material, serves as an accumulator of moisture that is able to collect the steam released in the gas flow during the expiratory phase when the gas is not transferred to the patient.

Later, during the subsequent inspiratory phase, the preheated gas collects the steam instantaneously produced by the evaporation chamber 1 a, (but doesn't get saturated), and flowing to the patient passes through the exchanger element 5, collecting the moisture accumulated during the expiratory phase. The exchanger element 5 is then dried and made so greedy of moisture for the next phase of steam accumulation.

This also avoids the risk of saturation/occlusion of the exchanger element 5. So the air passing through the inspiratory limb I of the respiratory circuit 20 downstream to the exchanger element 5 is enriched to the level of humidity set up by the regulator 4 and maintained, through the thermoregulation of the limb, until the patient is reached.

In the ideal situation the amount of moisture reaching the patient corresponds precisely to the required amount that was set on the regulator 4. Based on the different initial conditions of ventilation, parameters of a flow of gas and the frequency of inspiration/expiration cycle, the amount of moisture to be supplied to the respiratory circuit 20 can be adjusted accordingly, at the discretion of the physician, plus or minus (inspiration gas saturated or not saturated).

The present invention has notable advantages and achieves the purposes described above.

The humidifier according to the invention allows the physician to actually adjust the amount of moisture delivered to the patient through the inspiratory gas.

Further, due to the small amounts of water to be vaporized the humidifier is extremely fast both when starting evaporation and when it's stopped. The resulting benefit is a rapid response to conditions changes and circumstances of alarm.

Another advantage is that the amount of water that must be provided to the patient through the respiratory gas flow is moved precisely to the evaporation chamber through the use of an efficient water dosing system.

Another advantage is that the all metered water is completely vaporized in the evaporation chamber.

The fact that there is no stagnant water in the evaporation chamber and neither in the inspiratory limb, reduces advantageously the risk of bacterial growth.

Another advantage related to the use of means for collecting and exchanging vaporized fluid is that in the respiratory circuit the entire amount of water vaporized is completely transferred.

A further advantage is that a fine adjustment of the dosing system allows to precisely determine the amount of vapor that is constantly transferred to the respiratory circuit.

Since each complete ventilation cycle consists of a phase of inspiration and a phase of expiration opposed one to another, the excess moisture not used during the inspiration phase is collected from the exchanger element 5 placed downstream. In this way the humidifier according to the present invention is able to provide the patient with a gas featuring moisture content almost constant at each ventilation cycle.

Further, advantageously with a not disposable evaporation chamber 1a, the self-sterilization of the chamber 1a itself is obtained through the raising of the steam temperature.

### List of referenced signs

- 100: humidifier
- 1: evaporator
- 1a: evaporation chamber
- 1b: heated channels
- 1c: heating cartridge
- 1d: upper chamber
- 2: fluid reservoir
- 30: dosing means
- 3: motion movement mechanism
- 3a: first connecting pipe
- 3b: second connecting pipe
- 3c: upstream valve
- 3d: downstream valve
- 3e: deformable member
- 4: regulator
- 5: humidity exchanger
- 6: connector
- 6a: temperature probe port
- 6b: diffusion nozzle
- 6c: connector port
- 7: first section upstream
- 8: second section downstream
- 8a: port
- 9: connecting tube
- 20: respiratory circuit
- I: inspiratory limb
- E: expiratory limb
- P: patient
- V: ventilator

## Claims

1. A humidifier for humidifying a gas in a respiratory circuit (20), comprising
- an evaporator (1) arranged to receive a defined quantity of fluid and vaporize said defined quantity of fluid, the evaporator (1) being positioned outside an inspiratory limb (I) of said respiratory circuit (20),
- a fluid reservoir (2) in fluid communication with said evaporator (1), and
- dosing means (30) to feed and dose said fluid from the reservoir (2) to the evaporator (1),
- connecting means (6, 9) to connect the evaporator (1) to the respiratory circuit (20) for supplying the vaporized fluid to the respiratory circuit (20), **characterized in that** it comprises an accumulator for collecting vaporized fluid and exchanging it with a gas flow flowing along the respiratory circuit (20).

2. A humidifier as claimed in claim 1, **characterized in that** said accumulator comprises a humidity exchanger (5) arranged in the respiratory circuit (20).

3. A humidifier as claimed in claim 2, **characterized in that** said connecting means comprise a vapour diffusion nozzle (6b) designed to supply the vaporized fluid directly to the humidity exchanger (5).

4. A humidifier as claimed in claim 3, **characterized in that** said connecting means comprise a connector (6) linked to the evaporator (1) and integrating said nozzle (6b), said connector (6) having a tubular body extending inside the respiratory circuit (20), and said nozzle (6b) being arranged at a distal end of said tubular body facing the humidity exchanger (5).

5. A humidifier as claimed in any of claims 2 to 4, **characterized in that** the humidity exchanger (5) is an HME or a filter-HME.

6. A humidifier for humidifying a gas in a respiratory circuit (20), comprising
- an evaporator (1) arranged to receive a defined quantity of fluid and vaporize said defined quantity of fluid, the evaporator (1) being positioned outside an inspiratory limb (I) of said respiratory circuit (20),
- a fluid reservoir (2) in fluid communication with said evaporator (1), and
- dosing means (30) to feed and dose said fluid from the reservoir (2) to the evaporator (1),
- connecting means (6, 9) to connect the evaporator (1) to the respiratory circuit (20) for supplying the vaporized fluid to the respiratory circuit (20), **characterized in that** said connecting means comprise a vapour diffusion nozzle (6b) designed to supply the vaporized fluid directly inside the inspiratory limb (I).

7. A humidifier as claimed in claim 4 or 6, **characterized in that** said vapour diffusion nozzle (6b) comprises a last portion extending coaxial to said tubular body of the connector (6) and/or coaxial to said inspiratory limb (I).

8. A humidifier as claimed in any of the preceding claims, **characterized in that** said dosing means (30) is designed to withdraw the defined quantity of fluid from the fluid reservoir (2) and transfer it to the evaporator (1), said defined quantity of fluid being fully vaporized in the evaporator (1).

9. A humidifier as claimed in claim 8, **characterized in that** it comprises a regulator (4) for achieving a defined humidification level of the gas in the respiratory circuit (20), said regulator (4) interacting with the dosing means (30) instructing said dosing means (30) to withdraw the defined quantity of fluid from the fluid reservoir (2) to be vaporized inside the evaporator (1).

10. A humidifier as claimed in any of the preceding claims, **characterized in that** the dosing means (30) comprises a motion movement mechanism (3) deforming a deformable member (3e) connected between the fluid reservoir (2) and the evaporator (1), said deformation of the deformable member (3e) changing the volume inside the deformable member (3e), wherein a decrease of the volume in the deformable member (3e) increases the pressure inside the deformable member (3e) closing an upstream valve (3c) and opening of a downstream valve (3d) resulting in a transfer of the defined quantity of fluid to the evaporator (1), and an increase of the volume in the deformable member (3e) decreases the pressure inside the deformable member (3e) causing the closure of the downstream valve (3d) and the opening of the upstream valve (3c) withdrawing the defined quantity of fluid from the fluid reservoir (2).

11. A humidifier as claimed in claim 10, **characterized in that** the motion movement mechanism (3) is moving eccentrically, where the lowering and raising of the mechanism (3) onto the deformable member (3e) causes its deformation.

12. A humidifier as claimed in claim 10 or 11, **characterized in that** the deformable member (3e) is a tube.

13. A humidifier as claimed in any of the preceding claims, **characterized in that** the fluid is water.
